# EUROPEAN PATENT APPLICATION

(11) **EP 4 279 057 A1**
(43) Date of publication of application: **22.11.2023**
(21) Application number: 22177200.7
(22) Date of filing: 03.06.2022
(51) Int. Cl.: A61K 8/06, A61K 8/20, A61K 8/23, A61K 8/34, A61K 8/365, A61K 8/37, A61K 8/40, A61K 8/42, A61K 8/44, A61K 8/46, A61K 8/49, A61K 8/60, A61K 8/67, A61Q 19/00

(54) **COSMETIC OR PERSONAL CARE COMPOSITION COMPRISING A COMBINATION OF AW-LOWERING SUBSTANCES**

(30) Priority: 20.05.2022 DE 102022205087
(71) Applicant: Cosphatec GmbH, 20457 Hamburg (DE)
(72) Inventor: Schröder, Markus Sebastian, 22045 Hamburg (DE); Scholz, Jonas, 20459 Hamburg (DE); Nadarzynski, Alexandra, 22083 Hamburg (DE)
(74) Representative: Uexküll & Stolberg

(57) **Abstract**

The present invention relates to a cosmetic or personal care composition comprising at least three a_{w} (water activity)-lowering substances in an amount achieving an antimicrobial effect and resulting in a skin barrier-enhancing effect, wherein at least 1/3 of the total amount of the at least three a_{w}-lowering substances are moisturizing agents, and wherein the water activity of the cosmetic or personal care composition is no greater than 0.94. The present invention further relates to a cosmetic or personal care composition comprising at least three a_{w}-lowering substances and at least one antimicrobial substance in an amount achieving an additive or synergistic antimicrobial effect. The invention further relates to a method of inhibiting microbial growth in a cosmetic or personal care composition using at least three a_{w}-lowering substances or a combination of at least three a_{w}-lowering substances and at least one antimicrobial substance. The invention also relates to the use of said combinations for controlling microbial growth in a composition and resulting in a skin barrier-enhancing effect.

## Description

### Field of the Invention

The present invention relates to cosmetic or personal care compositions comprising at least three a_{w} (water activity)-lowering substances having water activity no greater than 0.94 effective for inhibiting microbial growth in the cosmetic or personal care compositions and for resulting in a skin barrier-enhancing effect. The invention further relates to cosmetic or personal care compositions further comprising at least one antimicrobial substance in an amount achieving an additive or synergistic antimicrobial effect.

The invention further relates to preservative composition blends comprising at least three a_{w}-lowering substances as well as a combination of at least three a_{w}-lowering substances and an antimicrobial substance.

Further, the invention relates to a method of inhibiting microbial growth in cosmetic and personal care compositions using at least three a_{w}-lowering substances or a combination of at least three a_{w}-lowering substances and one antimicrobial substance as well as a method of enhancing the effect of a user's skin barrier with said combinations.

The invention also relates to the use of said combinations for controlling microbial growth in a composition and resulting in a skin barrier-enhancing effect.

### Background of the invention

Most cosmetic or personal care products are ideal substrates for the development and survival of a large variety of microorganisms like bacteria, yeast and mold, since they often contain more than 50 wt% of water and many nutrients that facilitate their growth.

The associated change in color, odor and consistency of such products not only leaves the user feeling queasy, but can also lead to skin irritations, allergic contact dermatitis and infections, especially in the area of the mucous membranes.

The use of chemical preservatives should therefore not only improve the microbiological quality of cosmetic and personal care products, but also provide safety for consumers.

However, recent studies and reports indicate that this is not always the case when it comes to traditional chemical preservatives, such as imidazolidinyl urea or isothiazolinones which have recently attracted negative attention due to their allergenic potential. In addition, official regulations and obligations on ingredient declaration are becoming more extensive and more detailed.

So the consumer demand for alternative preservatives as well as strategies to avoid or reduce traditional preservatives is steadily increasing.

One possibility to replace or lower the amount of traditional preservatives is to focus on the factors that can negatively influence the growth of microorganisms, such as a low redox potential, an acidic or basic pH value or a low water activity. These factors can act as hurdles for microorganisms, wherein the concept of the so called "hurdle technology" finds its origin in the preservation of food.

For cosmetic or personal care products the challenge here is primarily the area of application, namely human skin. To avoid a negative impact of hurdle technology strategies on skin integrity, measures to achieve antimicrobial efficacy have to be precisely balanced. Water activity is an ideal hurdle factor that can be focussed on for cosmetic or personal care compositions via addition of water-binding substances.

EP 0 755 674 B1 relates to a composition containing ascorbic acid, wherein a polyol is used to reduce the water activity in the composition to 0.85 in combination with a structuring agent preventing the degradation of the ascorbic acid.

EP 0 779 071 B1 relates to a water/oil/water triple emulsion wherein the use of a polyol in an amount from 35% to 90% results in an aqueous phase with a water activity value of less than or equal to 0.85.

Unfortunately, most antimicrobial substances only show good antimicrobial activity against specific microorganisms, for example bacteria only, but no or very weak activity against yeast and mold. As a result, several of these antimicrobials are usually combined, which leads to an increased total concentration of these substances.

So there is still a need to find strategies to preserve cosmetic and personal care compositions that give an adequate protection against the full spectrum of microorganisms without the use or with a lower amount of traditional preservatives, which are coming under increasing criticism from consumers.

Furthermore, there is a demand for new systems that allow one to use antimicrobial substances in reduced concentrations.

Cosmetic formulators also face the challenge of developing new solutions to control microbial growth in cosmetic or personal care compositions that do not have a negative impact on the skin, but on the contrary ideally offer benefits for the skin.

Summarized, there is a need to find new solutions for avoiding traditional preservatives in cosmetic or personal care compositions in high concentrations that are safe for consumers and having no negative impact on the skin.

### Summary of the invention

According to the present invention the above problems are solved by a cosmetic or personal care composition comprising at least three a_{w}-lowering substances in a total amount of at least 10 wt%, relative to the total weight of the composition, achieving an antimicrobial effect and resulting in a skin barrier-enhancing effect, wherein at least 1/3 of the total amount of the at least three a_{w}-lowering substances are moisturizing agents, and wherein the water activity of the cosmetic or personal care composition is no greater than 0.94.

It has been surprisingly found that cosmetic or personal care compositions comprising at least three a_{w}-lowering substances not only show an antimicrobial effect, but also a skin barrier-enhancing effect when applying them regularly.

The above problems are also solved by a cosmetic or personal care composition comprising a combination of at least three a_{w}-lowering substances and at least one antimicrobial substance, wherein at least 1/3 of the total amount of the at least three a_{w}-lowering substances are moisturizing agents, and wherein the water activity of the cosmetic or personal care composition is no greater than 0.94.

It has been surprisingly found that cosmetic or personal care compositions comprising the combination of at least three a_{w}-lowering substances and at least one antimicrobial substance show an additive or even a synergistic antimicrobial effect. So the microbial growth can be controlled by a_{w}-lowering substances and even enhanced when adding low amounts of at least one antimicrobial substance.

The invention also provides preservative composition blends comprising at least three a_{w}-lowering substances as well as a combination of at least three a_{w}-lowering substances and at least one antimicrobial substance.

Furthermore, the invention provides a method of inhibiting microbial growth in cosmetic or personal care compositions as well as a method of enhancing the effect of a user's skin barrier as defined in more detail below.

Finally, the present invention provides the use of said combinations for controlling microbial growth and resulting in a skin barrier-enhancing effect.

Further aspects of the invention are disclosed in the appended claims. In certain aspects of the invention, embodiments consist of the components disclosed herein.

### Detailed description of the invention

According to the present invention cosmetic or personal care compositions are provided which comprise at least three a_{w}-lowering substances in a total amount of at least 10 wt%, relative to the total weight of the composition, achieving an antimicrobial effect and resulting in a skin barrier-enhancing effect, wherein at least 1/3 of the total amount of the at least three a_{w}-lowering substances are moisturizing agents, and wherein the water activity of the cosmetic or personal care composition is no greater than 0.94.

Furthermore, cosmetic or personal care compositions are provided that comprise at least three a_{w}-lowering substances and at least one antimicrobial substance in an amount achieving an additive or synergistic antimicrobial effect.

The term "antimicrobial substance" is intended to mean a substance that is capable of preventing or reducing the growth or proliferation of and/or killing microorganisms such as bacteria, yeast and mold and is understood by the person skilled in the art.

In the context of the present application, "antimicrobial effect" means the effect of an antimicrobial substance of reducing the growth or proliferation of and/or killing microorganisms such as bacteria, yeast and mold in a cosmetic or personal care composition. The antimicrobial effect can be determined as described in the examples section.

In the context of the present application, an "additive antimicrobial effect" means an antimicrobial effect of the combination of the at least three a_{w}-lowering substances and the at least one antimicrobial substance that is the sum of the antimicrobial effects of each of the ingredients when used alone. In the context of the present application, a "synergistic antimicrobial effect" means an antimicrobial effect of the combination of the at least three a_{w}-lowering substances and the at least one antimicrobial substance that is larger than the antimicrobial effect of the sum of the effects of each of the ingredients when used alone. In concrete terms, this means that the reduction of the number of viable bacteria, yeast and/or mold is larger for the combination of the at least three a_{w}-lowering substances and the at least one antimicrobial substance compared to the sum of the reduction of the number of viable bacteria achieved by the addition of the identical amounts of the individual components.

The term "a_{w}-lowering substance" is intended to mean an agent which can lower the water activity of a cosmetic or personal care composition by binding water. Water activity therefore does not reflect the total water content of a cosmetic or personal care composition, but the amount of "freely available", unbound water. This characteristic is in principle known in the art and can be determined by determining the change in water activity associated with the use of a respective agent. The water activity according to the present invention is determined by a dew point hygrometer. According to this method the temperature at which a dew is formed on a clean surface is directly related to the vapor pressure of the air. Therefore, a mirror is placed over a closed sample chamber and is cooled until the dew point temperature is measured by means of an optical sensor. This dew point temperature is then used to find the relative humidity of the chamber using psychometrics charts. The accuracy of this method lies in 0.003 a_{w}.

In the context of the present application, a "skin barrier-enhancing effect" means the effect of a substance or a combination of substances of positively influencing the skin balance, such as an increase of the stratum corneum (SC) hydration and/or a decrease of the TEWL (transepidermal water loss) .

The term "TEWL (transepidermal water loss)" is understood by a person skilled in the art as the measure of the diffusion rate of water vapor through the epidermis of human skin. It measures the specific mass flow of water vapor per area and unit time through the skin. TEWL is usually expressed in units of g/m²/h or µg/mm²/h. The measurement of TEWL is a method commonly used in dermatological research to characterize the membrane function of the skin.

The term "stratum corneum (SC) hydration" is understood by a person skilled in the art as the measurement of skin hydration using a corneometer, wherein the results are usually compared to the baseline (untreated) skin. SC hydration is usually determined by measuring the electrical capacitance of the skin surface expressed in arbitrary units (a.u.).

### a_{w}-lowering substance

The cosmetic or personal care composition of the present invention comprises at least three a_{w}-lowering substances in a total amount of at least 10 wt%, relative to the total weight of the composition.

An a_{w}-lowering substance of the present invention is a water-binding substrate able to reduce the amount of "freely available", unbound water in a cosmetic or the personal care composition. The at least three a_{w}-lowering substances of the present invention are different substances, preferably at least one of the three different a_{w}-lowering substances is a moisturizing agent.

The cosmetic or personal care composition may comprise the at least three a_{w}-lowering substances in a total amount of at least 12 wt%, relative to the total weight of the composition.

In a preferred embodiment of the invention, the cosmetic or personal care composition contains at least three a_{w}-lowering substances in a total amount of at least 15 wt%, relative to the total weight of the composition.

In a particularly preferred embodiment of the present invention the total amount of the at least three a_{w}-lowering substances is higher than 25 wt%, preferably higher than 30 wt%, in particular higher than 35 wt%, relative to the total weight of the composition.

In a preferred embodiment of the invention, the cosmetic or personal care composition contains the at least three a_{w}-lowering substances in a total amount of not more than 60 wt%, preferably not more than 50 wt%, in particular not more than 40 wt%.

The maximum amount of a_{w}-lowering substances may depend on the total amount of water used in the cosmetic or personal care composition. In the case of water-based formulations, the total amount of a_{w}-lowering substances is preferably higher than for emulsions having a significant proportion of nonaqueous components. For example, in a shower gel, the total amount of the at least three a_{w}-lowering substances may be in the range between 50 to 60 wt%. On the other hand, in an emulsion, the total amount of the at least three a_{w}-lowering substances may be in the range between 40 to 50 wt%. In rather "dry" formulations, such as scrubs or clay facial masks, the total amount of the at least three a_{w}-lowering substances may be lower than 40 wt%, preferably lower than 30 wt%.

An a_{w}-lowering substance of the present invention is preferably a substance with a maximum molecular weight of 600 g/mol. In a preferred embodiment of the invention, the molecular weight of an a_{w}-lowering substance is at most 500 g/mol. In a particularly preferred embodiment, the molecular weight of an a_{w}-lowering substance of the present invention is at most 400 g/mol.

Alternatively or simultaneously, the a_{w}-lowering substance of the present invention preferably has a water solubility according to OECD Guideline 105 of at least 1%. The OECD Guideline 105 for the testing of Chemicals was adopted in 1981 and revised by the Council on July 27 1995. It addresses the determination of the solubility in water of essentially pure substances which are stable in water and not volatile. The determination of the water activity according to the present invention can be found in the OECD Guideline 105 in the chapter "Description of the methods" (Sections 7 to 26). In a preferred embodiment of the invention, the water solubility of an a_{w}-lowering substance is at least 3%. In a particularly preferred embodiment of the present invention the water solubility of an a_{w}-lowering substance is at least 5%.

The at least three a_{w}-lowering substances of the present invention are selected from the group consisting of C₃ to C₆ diols, C₄ to C₆ alditols, glycerol, C₂ to C₆ aldoses and/or ketoses, disaccharides, amino acids and/or salts thereof, peptides, betaine, carnitine, N-containing amino acid analogues like creatine, ectoine and/or L-pyrrolidone carboxylate and/or salts thereof, vitamin B or vitamin C, vitamin B or vitamin C derivatives and/or precursors, urea, allantoine, caffeine, inorganic salts, carboxylic acids and/or derivatives, hydroxy acids, keto acids, sulfate-containing surfactants, sugar surfactants, amino acid-based surfactants, polyglycerol-based surfactants and/or mixtures thereof.

In the following discussion of preferred embodiments, reference is also briefly made to "the a_{w}-lowering substance" when describing chemical species that represent specific (preferred) choices for said substance(s). However, this is not to be understood as meaning that a single a_{w}-lowering substance is used but should be read in the context of the teaching of the present invention that at least three of said a_{w}-lowering substances are used. Alternatively, the following disclosures refer to "one of the at least three a_{w}-lowering substances" to describe preferred a_{w}-lowering substances according to the present invention.

In a preferred embodiment of the invention, the a_{w}-lowering substance may be a C₃ to C₆ vicinal 1,2-diol. In a vicinal diol, the two hydroxyl groups occupy in a vicinal position, that is, they are attached to adjacent carbon atoms. The C₃ to C₆ 1,2-diol may be 1,2-propanediol, 1,2-butanediol, 1,2-pentanediol or 1,2-hexanediol, preferably 1,2-pentanediol. In an alternative embodiment the a_{w}-lowering substance may be a C₃ to C₆ 1,3-diol, preferably 1,3-propanediol.

In another preferred embodiment of the invention, the a_{w}-lowering substance may be an C₄ to C₆ alditol, which are also known as sugar alcohols. In an alditol with a linear, noncyclic structure a hydroxy group is attached to each carbon atom. In a particularly preferred embodiment, the C₄ to C₆ alditol is selected from the group consisting of mannitol, xylitol, sorbitol, threitol, erythritol and arabitol.

In another preferred embodiment of the invention, the a_{w}-lowering substance may be glycerol.

The a_{w}-lowering substance of the present invention may also be an aldose and/or a ketose with a number of carbon atoms from 2 to 6, preferably with 5 or 6 carbon atoms. In a particularly preferred embodiment, the a_{w}-lowering substance may be glucose, fructose or galactose.

In another preferred embodiment of the invention, the a_{w}-lowering substance is a disaccharide, preferably extracted from plants, isolated from yeast or fungi, extracted from algae or through enzymatic breakdown of starch. In a particularly preferred embodiment, the disaccharide is cellobiose, isomaltose, lactose, maltose, melibiose, neotrehalose, saccharose or trehalose.

In another preferred embodiment of the invention, the at least one a_{w}-lowering substance is an amino acid or an amino acid derivative like peptides. In another preferred embodiment of the invention, the a_{w}-lowering substance is a salt or a complex of an amino acid. The a_{w}-lowering substances of the present invention include amino acids like proteinogenic amino acids and non-proteinogenic amino acids as well as their salts and complexes. Furthermore, the a_{w}-lowering substance may be a quaternary ammonium compound like betaine or carnitine as well as N-containing amino acid analogues like creatine, ectoine or PCA (pyrrolidone carboxylic acid).

In another preferred embodiment of the invention, the a_{w}-lowering substance is an organic acid or a salt of an organic acid or a complex of an organic acid.

Said organic acid(s) of the present invention is selected from the group consisting of monocarboxylic acids, dicarboxylic acids, tricarboxylic acids, hydroxy acids, keto acids, and mixtures thereof.

Hydroxy acids of the present invention include alpha-hydroxy acids, beta-hydroxy acids and polyhydroxy acids as well as esters thereof, keto acids include alpha-, beta- and gamma-keto acids.

The a_{w}-lowering substance of the present invention may also be a vitamin, a vitamin derivative, a vitamin precursor or a salt or a complex thereof, preferably vitamin B or vitamin C derivatives, wherein vitamin C derivatives are particularly preferred. These may include magnesium ascorbyl phosphate, sodium ascorbyl phosphate, ascorbyl glucoside, 3-0-ethyl ascorbic acid, niacinamide and panthenol.

In a preferred embodiment of the invention, the a_{w}-lowering substance may be a metabolite or a nucleic acid degradation product like urea or allantoine.

In another preferred embodiment of the invention, purine alkaloids like caffeine or their salts or their complexes are used as a_{w}-lowering substances.

In a preferred embodiment of the invention, the a_{w}-lowering substance may be a surfactant. Surfactants include sulfate-containing surfactants, sugar surfactants, amino acid-based surfactants, polyglycerol-based surfactants and/or mixtures thereof. These a_{w}-lowering substance may be sodium lauryl sulfate, sodium lauryl sulfoacetate, ammonium lauryl sulfate, sodium cocosulfate, sodium lauryl glutamate, sodium cocoglutamate, sodium N-laurolyalanine, sodium cocolisethionate, sodium lauroylisethionate, sodium cocoyl methyl isethionate, lauryl glucoside, decylglucoside, cocoglucoside, polyglyceryl-3-laurate, caprylyl/capryl glucoside, disodium cocoyl glutamate or polyglyceryl-4-caprate.

The a_{w}-lowering substance of the invention may be an inorganic salt. Inorganic salts may include MgSO₄, NaSO₄, NaCl, KCl, LiCl and NH₄Cl as well as mixtures thereof.

In a preferred embodiment of the invention, one of the at least three a_{w}-lowering substances is an organic acid and/or a salt thereof with a water solubility according to OECD Guideline 105 of at least 1%. In a particularly preferred embodiment the a_{w}-lowering substance may be an organic acid and/or a salt thereof with a water solubility according to OECD Guideline 105 of at least 3%, preferably of at least 5%. In an alternative embodiment of the present invention one of the at least three a_{w}-lowering substances may be a C₄ to C₆ alditol and/or a vitamin B derivative and/or a salt thereof with a water solubility according to OECD Guideline 105 of at least 1%, preferably of at least 3%, in particular of at least 5%.

One of the at least three a_{w}-lowering substances of the present invention may be selected from the group consisting of lactic acid, citric acid, mannitol, xylitol, sorbitol, threitol, erythritol, arabitol, nicotinic acid, niacinamide and/or mixtures therof including suitable salts of the aforementioned acids.

According to the present invention, 1/3 of the total amount of the at least three a_{w}-lowering substances are moisturizing agents. A moisturizing agent of the present invention is a substance that is added to the cosmetic or personal care composition to improve skin moisture parameters, preferably to reduce TEWL and/or increase SC hydration. This may result in a smoother appearance of the user's skin.

In a preferred embodiment, 2/3 of the total amount of the at least three a_{w}-lowering substances are moisturizing agents. In a particularly preferred embodiment, the total amount of the a_{w}-lowering substances are moisturizing agents.

Moisturizing agents of the present invention may be selected from the group consisting of glycerol, propylene glycol, 1,3-propanediol, butylene glycol, 1,2-pentanediol, 1,2-hexanediol, lactic acid, citric acid, ascorbic acid, glycolic acid, niacinamide, amino acids, PCA, ectoine, caffeine, betaine, triacetine, urea, panthenol, sorbitol, xylitol, maltitol, mannitol, erythritol, mono- and disaccharides such as fructose, glucose, lactose, sucrose, inositol, trehalose, and/or salts thereof and/or mixtures thereof.

### Antimicrobial substance

The cosmetic or personal care composition of the invention may further comprise at least one antimicrobial substance.

The at least one antimicrobial substance may be present in an amount of 0.01 to 6 wt%, relative to the total weight of the composition. Alternatively, a combination of two or more antimicrobial agents - or even a preservation blend - can be used, wherein the total amount of these antimicrobial substances preferably does not exceed an amount of 2 wt%, relative to the total weight of the composition.

In a preferred embodiment the at least one antimicrobial substance is present in an amount of 0.1 to 1 wt%, relative to the total weight of the composition. In a particularly preferred embodiment of the invention the cosmetic or personal care composition does not contain more than 0.50 wt% of at least one antimicrobial substance, relative to the total weight of the composition.

The at least one antimicrobial substance according to the present invention may be one single compound, a mixture of two or more different compounds or a prefabricated blend consisting of compounds described herein below. The at least one antimicrobial substance may also be an extract from a natural source.

It is to be understood that the at least one antimicrobial substance of the invention is different from the at least three a_{w}-lowering substances. Thus, for example, if 1,3-propanediol is chosen as the first a_{w}-lowering substance, 1,3-propanediol is not also chosen as antimicrobial substance. However, the antimicrobial substance may be another diol that shows antimicrobial activity. If a substance used as an a_{w}-lowering substance according to the invention also has an antimicrobial effect (other than by influencing a_{w}), for the purpose of determining the amounts in which the respective active components are used, said substance will be included in the group of a_{w}-lowering substances present in the compositions of the present invention.

In a preferred embodiment of the invention, the antimicrobial substance is an amphiphilic compound. Alternatively or simultaneously the antimicrobial substance may be capable of weakening the outer shell of the cell membrane structure in microorganisms.

The at least one antimicrobial substance may be selected from the group consisting of C₃ to C₁₀ diols, glyceryl esters of a C₈ to C₁₂ carboxylic acid, C₆ to C₁₀ hydroxamic acids, bi-(hydroxyphenyl) compounds, unsaturated and/or aromatic C₆ to C₁₀ carboxylic acids and/or salts thereof, C₄ to C₁₀ oxocarboxylic acids and/or salts thereof and/or mixtures thereof.

In a preferred embodiment of the invention, the at least one antimicrobial substance is a C₃ to C₁₀ diol.

The C₃ to C₁₀ diol can have any substitution pattern known to a person skilled in the art. The diol may be branched or linear.

In a preferred embodiment of the invention, the C₃ to C₁₀ diol is a 1,2-(vicinal) diol or a 1,3-diol. The C₃ to C₁₀ vicinal diol may be 1,2-propanediol, 1,2-butanediol, 1,2-pentanediol, 1,2-hexanediol, 1,2-heptanediol, 1,2-octanediol, 1,2-nonanediol or 1,2-decanediol.

In a preferred embodiment of the invention, the antimicrobial substance is a C₅ to C₇ vicinal diol. In a particularly preferred embodiment, the antimicrobial substance is 1,2-pentanediol, or 1,2-hexanediol.

In another preferred embodiment of the invention, the antimicrobial substance is a C₃ to C₇ 1,3-diol, preferably 1,3-propanediol.

The antimicrobial substance of the present invention may also be a glyceryl ester of a C₈ to C₁₂ carboxylic acid.

In a preferred embodiment of the invention, the antimicrobial substance is a glyceryl monoester. The antimicrobial substance may be a glyceryl monoester of a linear C₈ to C₁₂ carboxylic acid. In a particularly preferred embodiment, the antimicrobial substance is glyceryl caprylate, glyceryl laurate or glyceryl undecylenate.

The antimicrobial substance may also be a C₆ to C₁₀ hydroxamic acid. A hydroxamic acid is an organic compound bearing the functional group RC(O)N(OH)R', with R and R' as organic residues and CO as a carbonyl group. In a preferred embodiment of the invention, the antimicrobial substance is a C₆ to C₁₀ hydroxamic acid with a saturated alkyl chain. In a particularly preferred embodiment, the antimicrobial substance is caprylhydroxamic acid.

The antimicrobial substance according to the present invention may also be at least one bi-(hydroxyphenyl) compound. In a preferred embodiment of the invention, the antimicrobial substance is at least one bi-(hydroxyphenyl) compound, wherein each phenyl ring is substituted with one allyl group. The antimicrobial substance may be Magnolol and/or Honokiol. These are polyphenolic containing compounds in which Honokiol is an isomer of Magnolol. Honokiol and Magnolol can be isolated or purified from Magnolia extracts (e.g., flower, bark, and seed cone extracts) or other extracts that include such compounds by standard techniques.

In a preferred embodiment of the invention, the antimicrobial substance is an extract comprising Magnolol and Honokiol. The extract may comprise Magnolol and Honokiol in a ratio of approximately 1:1.

The extract may be a Magnolia extract. The Magnolia extract can be obtained or derived from a variety of sources from a Magnolia plant (e.g., flower, bark, seed cone, etc.). The Magnolia extract can be obtained from the species within the *Magnoliaceae* family. Non-limiting examples of these species include *Magnolia acuminata, Magnolia ashei, Magnolia biondii, Magnolia cylindrica, Magnolia cambellii, Magnolia denudata, Magnolia fraseri, Magnolia grandiflora, Magnolia hypoleuca, Magnolia kobus, Magnolia liliflora, Magnolia loegneri, Magnolia macrophylla, Magnolia officinalis, Magnolia pyramidata, Magnolia sargentiana, Magnolia seiboldii, Magnolia soulangiana, Magnolia sprengeri, Magnolia stellata, Magnolia tripetala, Magnolia virginiana, Magnolia zenii, and Michelia figo.*

In a particularly preferred embodiment, the antimicrobial substance is a *Magnolia officinalis* bark extract. The extract may be obtained via extraction with supercritical CO₂. Ethanol may be used at the end of the extraction process for purification.

In a preferred embodiment of the invention, the antimicrobial substance is an unsaturated and/or aromatic C₆ to C₁₀ carboxylic acid selected from the group consisting of anisic acid, sorbic acid, cinnamic acid, ferulic acid and/or mixtures thereof and/or a C₄ to C₁₀ oxocarboxylic acid like levulinic acid and/or acetoacetic acid.

In a particularly preferred aspect of the invention, the composition of the present invention comprises an antimicrobial substance selected from 1,2-pentanediol, 1,2-hexanediol, 1,3-propanediol, sodium levulinate, levulinic acid, sodium anisate, glyceryl caprylate, caprylhydroxamic acid and/or a *Magnolia officinalis* bark extract or mixtures thereof.

According to another aspect, the cosmetic or personal care compositions of the present invention do not include traditional preservatives as antimicrobial substances as listed in the cosmetic regulations No. 1223/2009 from November 30, 2009, appendix 5, such as phenoxyethanol, benzoic acid, sodium benzoate, propionic acid and salts thereof, salicylic acid and salts thereof, formic acid and salts therof, undecylenic acid and salts thereof, dichlorobenzyl alcohol, triclosan, chloroxylenol, imidazolidinyl urea, polyaminopropyl biguanidine, climbazole, benzyl alcohol, chlorhexidine, phenoxyisopropanol, diazolidinyl urea, dimethyl oxazolidine, chlorphenesin, methylisothiazolinone, parabenes and/or ethyl lauryl arginate.

### Cosmetic or personal care composition of the invention

The cosmetic or personal care composition of the invention may be used for topical applications. The composition of the present invention is suitable for, but not limited to, formulations for use on lips, sculp or skin.

In a preferred embodiment of the invention, the cosmetic or personal care composition comprises at three a_{w}-lowering substances in a total amount of at least 10 wt%, relative to the total weight of the composition.

In another preferred embodiment of the invention, the cosmetic or personal care composition comprises at three a_{w}-lowering substances in a total amount of at least 10 wt% and preferably at least one antimicrobial substance in an amount of 0.01 to 6 wt%, relative to the total weight of the composition.

Cosmetic or personal care composition are particularly well suited when they contain the at least three a_{w}-lowering substances in a total amount of at least 12 wt%, preferably in in a total amount of at least 15 wt%, relative to the total weight of the composition. In a particularly preferred embodiment of the present invention, the total amount of the at least three a_{w}-lowering substances is higher than 25 wt%, preferably higher than 30 wt%, in particular higher than 35 wt%, relative to the total weight of the composition.

In an alternative embodiment of the invention, the cosmetic or personal care composition further comprises an antimicrobial substance in an amount of 0.05 to 2 wt%, in a preferred embodiment in an amount of 0.1 to 1 wt%, relative to the total weight of the composition. Cosmetic or personal care composition are particularly preferred when they do not contain more than 0.50 wt% of at least one antimicrobial substance.

In a preferred embodiment of the invention, the water content of the cosmetic or personal care is 15 to 90 wt%, relative to the total weight of the composition.

Cosmetic or personal care compositions of the invention are preferred when they have a water activity no greater than 0.92. In a preferred embodiment, the cosmetic or personal care composition may have a water activity no greater than 0.85. In a particularly preferred embodiment of the invention the water activity of the cosmetic or personal care composition is no greater than 0.80.

In general, the cosmetic or personal care compositions of the present invention can be formulated over a broad range of pH values, such as from 3 to 8. In a preferred embodiment the pH can be in the range from 4 to 8, particularly preferred in the range from 5 or 5.5 to 7.

The cosmetic or personal care composition can be comprised in a cosmetic carrier (e.g., an emulsion, cream, lotion, solution, anhydrous base, gel, ointment, etc.). The carrier may be water-based, oil-based, or emulsion-based, preferably emulsion-based.

In embodiments where the carrier is emulsion-based, the composition may be in the form of a water-in-oil, an oil-in-water, a water-in-oil-in-water or an oil-in-water-in-oil emulsion, preferably an oil-in-water (o/w) emulsion.

The at least three a_{w}-lowering substances may be present in the cosmetic or personal care composition in an amount effective to reduce the number of viable/live bacteria, yeast and/or mold such as *Enterobacter gergoviae, Pseudomonas aeruginosa, Staphylococcus aureus, Escherichia coli, Candida albicans,* and *Aspergillus brasiliensis.* In a preferred embodiment of the invention the reduction of these bacteria, yeast and/or molds is also achieved by a cosmetic or personal care composition that comprises at least three a_{w}-lowering substances and at least one antimicrobial substance.

The at least three a_{w}-lowering substances or the combination of at least one antimicrobial substance and at least three a_{w}-lowering substances may be present in the composition in an amount effective to achieve an 'A or B criteria pass' for bacteria, yeast and mold (e.g., *Pseudomonas aeruginosa, Staphylococcus aureus, Escherichia coli, Candida albicans,* and *Aspergillus brasiliensis*) in accordance with the pass acceptance criteria outlined in ISO 11930.

The at least three a_{w} (water activity)-lowering substances are present in the composition in an amount effective to achieve at least one of the following criteria:
- no increase in the number of viable/live molds and yeasts against the value obtained for the inoculum at a time point of 14 days after inoculation of the composition and/or
- at least a 3 log reduction in the number of viable/live gram-negative bacteria against the value obtained for the inoculum at a time point of 14 days after inoculation of the composition.

The combination of at least one antimicrobial substance and at least three a_{w}-lowering substances may be present in the cosmetic or personal composition in an amount effective to achieve a maximum achievable log reduction in the number of viable microorganisms on day 7.

The cosmetic or personal care composition of the invention may be provided in any form suitable for topical application to the skin, lips or sculp. The cosmetic or personal care composition of the invention may be delivered and/or applied to the skin via any of the conventional formulations known to those skilled in the art. Typical formulation types of the present invention are liquids, creams, lotions, milks, mousses, gels, sprays, serum, foams, aerosols, and ointments.

The cosmetic and personal care composition of the invention will generally further comprise other ingredients or excipients which will be well known to those skilled in the art.

The composition of the invention may further comprise ingredients such as chelating agents, lubricants, solvents, water repellents, antioxidants, UV absorbers, anti-irritants, trace metals and/or structuring agents.

The compositions of the invention may further comprise one or more perfumes and/or colouring agents and/or pigments.

The composition of the invention may consist of the components as disclosed herein.

The use of the word "a" or "an" when used in conjunction with the terms "comprising," "including," "having," or "containing," or any variations of these terms, in the claims and/or the specification may mean "one," but it is also consistent with the meaning of "one or more," "at least one," and "one or more than one." Also, the reference to the definite article "the" does not necessarily mean that a single chemical species is used, as discussed, for example, hereinabove with regard to the a_{w}-lowering substance(s).

### Preservative composition blend

In another aspect of the present invention, a preservative composition blend for inhibiting microbial growth in a cosmetic or personal care composition is provided. The preservative composition blend is a prefabricated mixture of a combination of at least three a_{w}-lowering substances. The preservative composition blend of the present invention may further compromise at least one antimicrobial substance. The preservative composition blend of the present invention may be in form of a powder, a liquid concentrate or a granulate.

### Skin barrier-enhancing effect

The cosmetic or personal care composition of the present invention shows a skin barrier-enhancing effect, preferably in form of a decrease of the TEWL and/or an increase of the SC hydration.

In a preferred embodiment of the invention a decrease of the TEWL and an increase of the SC hydration can be detected on a user's skin after application of the cosmetic or personal care composition according to the present invention.

The at least three a_{w}-lowering substances or the combination of at least three a_{w}-lowering substances and the at least one antimicrobial substance may be present in the cosmetic or personal care composition in an amount effective to achieve a TEWL decrease of more than 5% and a SC hydration increase of more than 5% in a randomized forearm-controlled study (also discussed in the Examples section below).

### The method and use

In another aspect of the present invention, a method of inhibiting microbial growth in a cosmetic or personal care composition is provided.

The method of inhibiting microbial growth in a cosmetic or personal care composition comprises the steps of:
- introducing at least three a_{w}-lowering substances in a total amount of at least 10 wt%, relative to the total weight of the composition, achieving an antimicrobial effect,
   wherein at least 1/3 of the total amount of the at least three a_{w}-lowering substances are moisturizing agents, and
   wherein the water activity of the cosmetic or personal care composition is no greater than 0.94, and
- controlling the growth of at least one microorganism.

The method of the present invention may further compromise the step of:
- adding at least one antimicrobial substance in an amount achieving an additive or synergistic antimicrobial effect.

In a preferred embodiment, the at least one microorganism is from the genus Escherichia, Pseudomonas, Candida, Aspergillus and/or Staphylococcus, preferably *Escherichia coli, Pseudomonas aeruginosa, Candida albicans, Aspergillus brasiliensis* and/or *Staphylococcus aureus.*

In another aspect of the present invention, a method of enhancing the effect of a user's skin barrier when applying the cosmetic or personal care composition is provided.

The method of enhancing the effect of a user's skin barrier comprises the steps of:
- applying a cosmetic or personal care composition, preferably an o/w emulsion, according to the present invention or a cosmetic or personal care composition comprising a preservative composition blend of the present invention on a user's skin, preferably twice times daily, and
- obtaining a decrease of the TEWL and/or an increase of the SC hydration, preferably a decrease of the TEWL and an increase of the SC hydration.

In the methods according to the present invention, the at least three a_{w}-lowering substances are preferably C₃ to C₆ diols, C₄ to C₆ alditols, glycerol, C₂ to C₆ aldoses and/or ketoses, disaccharides, amino acids and/or salts thereof, peptides, betaine, carnitine, N-containing amino acid analogues like creatine, ectoine and/or L-pyrrolidone carboxylate and/or salts thereof, vitamin B or vitamin C, vitamin B or vitamin C derivatives and/or precursors, urea, allantoine, caffeine, MgSO₄, NaSO₄, NaCl, KCl, LiCl, NH₄Cl, carboxylic acids and/or derivatives, hydroxy acids, keto acids, sulfate-containing surfactants, sugar surfactants, amino acid-based surfactants, polyglycerol-based surfactants and/or mixtures thereof.

Furthermore, in the methods according to the present invention the at least one antimicrobial substance is preferably selected from C₃ to C₁₀ diols, glyceryl esters of a C₈ to C₁₂ carboxylic acid, C₆ to C₁₀ hydroxamic acids, bi-(hydroxyphenyl) compounds, unsaturated and/or aromatic C₆ to C₁₀ carboxylic acids and/or salts thereof, C₄ to C₁₀ oxocarboxylic acids and/or salts thereof and/or mixtures thereof.

In yet another aspect of the present invention, the use of a combination of at least three a_{w}-lowering substances in a total amount of at least 10 wt%, relative to the total weight of the composition, achieving an antimicrobial effect for controlling microbial growth and resulting in a skin barrier-enhancing effect is provided, wherein at least 1/3 of the total amount of the at least three a_{w}-lowering substances are moisturizing agents, and wherein the water activity of the cosmetic or personal care composition is no greater than 0.94.

In yet another aspect of the present invention, the use of a combination of at least three a_{w}-lowering substances in a total amount of at least 10 wt%, relative to the total weight of the composition, and at least one antimicrobial substance in an amount achieving an additive or synergistic antimicrobial effect for controlling microbial growth and resulting in a skin barrier-enhancing effect is provided, wherein at least 1/3 of the total amount of the at least three a_{w}-lowering substances are moisturizing agents, and wherein the water activity of the cosmetic or personal care composition is no greater than 0.94.

Otherwise, with regard to further aspects of the methods and uses of the present invention, the at least three a_{w}-lowering substances, the at least one antimicrobial substance and other potential ingredients and effects are as defined hereinabove for the cosmetic or personal care compositions according to the present invention.

The skilled person will understand that optional features of one embodiment or aspect of the invention may be applied, where appropriate, to other embodiments or aspects of the invention.

### Examples

The invention is illustrated by the following examples, which should not be considered as limiting the scope of the invention, but as illustrating it. The antimicrobial effect as well as the skin barrier effect of an o/w-emulsion should be described in detail, especially regarding the test methods and the evaluation of the test results.

### Antimicrobial effect

The testing method used is based on the European Pharmacopoeia (Ph. Eur.) chapter 5.1.3. (Ph. Eur. 8.6, 01/2011:50103) which is a commonly used method for testing the antimicrobial efficacy in cosmetic and personal care products.

To count the viable microorganisms in the inoculated products, the agar medium used for the initial cultivation of the respective microorganisms was used. A series of containers of the product to be examined, was inoculated each with a suspension of one of the test organisms to give an inoculum of 10⁵ to 10⁶ microorganisms per milliliter or per gram of the preparation. The volume of the suspension of inoculum did not exceed 1 per cent of the volume of the product. The suspension was thoroughly mixed to ensure homogeneous distribution. The inoculated product was maintained at 20-25°C, protected from light. A suitable sample was removed from each container, typically 1 ml or 1 g, at zero hour and at appropriate intervals according to the type of the product. The number of viable microorganisms by plate count or membrane filtration was determined. It was ensured that any residual antimicrobial activity of the product was eliminated.

The formulations were inoculated with one or more different microorganisms (*Escherichia coli (E. coli) Pseudomonas aeruginosa (P. aeruginosa), Staphylococcus aureus (S. aureus), Candida albicans* (C. *albicans*)*, Aspergillus brasiliensis (A. brasiliensis)).*

The bacterial strains used for the experiments were:
*Escherichia coli:* ATCC 8739
*Pseudomonas aeruginosa:* ATCC 9027
*Staphylococcus aureus:* ATCC 6538
*Candida albicans: ATCC 10231*
*Aspergillus brasiliensis: ATCC 16404*

Microbial count reductions of bacterial strains added to an oil-in-water emulsion were measured after 2, 7, 14 and 28 days. Synergistic antimicrobial activity is achieved if the observed microbial count reduction of a combination is higher than the sum of the reductions reached by the single components of the combination alone.

A_{w}-lowering substances in a concentration of 28 wt% were tested alone and in combination with 0.5 wt% of an antimicrobial substance containing a mixture of 60 wt% glyceryl caprylate and 40 wt% Magnolia Officinalis Bark Extract. The a_{w}-lowering substances were a combination of Cosphaderm^{®} Propanediol natural (propanediol), sodium lactate, Erylite^{®} (erythritol), betaine and Sodium PCA (sodium pyroglutamate) (Table 1). The antimicrobial substances were Cosphaderm^{®} GMCY (GMCY) and Cosphaderm^{®} Magnolia Extract 98 (Magnolia officinalis bark extract) (Table 2).

**Table 1:**

| a_{w}-lowering substance | INCI | Composition of combination [wt%] |
|---|---|---|
| | | 28 |
| Cosphaderm^{®} Propanediol natural | Propanediol | 8.0 |
| Sodium Lactate | Sodium Lactate | 8.0 |
| ERYLITE^{®} | Erythritol | 5.0 |
| Natural Betaine | Betaine | 5.0 |
| Sodium PCA | Sodium PCA | 2.0 |

**Table 2:**

| Antimicrobial substances | INCI | Ratio | Use concentration [wt %] |
|---|---|---|---|
| Cosphaderm^{®} GMCY (GMCY) / Cosphaderm^{®} Magnolia Extract 98 (ME) | Glyceryl Caprylate, Magnolia Officinalis Bark Extract | 60/40 | 0.5 |

For all tests the following oil-in-water emulsion was used (Table 3). The components of phase A and phase B were combined separately. For phase A, xanthan gum was dissolved in demineralised water under stirring at room temperature, and a_{w}-lowering substances as well as antimicrobial substances for some tests were added. Both phases were heated to 70 °C and Phase B was then added to phase A under stirring. The slightly cooled emulsion was homogenized for two minutes. Phase C was incorporated into the stirring emulsion at room temperature. The pH value was adjusted to 5.50 ± 0.05 with lactic acid (90%) or sodium hydroxide (30%) one day after preparation.

**Table 3:**

| **Phase** | **Ingredient** | **INCI** | **Quantity [wt%]** |
|---|---|---|---|
| A | Demineralised Water | Aqua | ad. 100 |
| | a_{w}-lowering substances¹ | - | 23.00 |
| | Antimicrobial substances² | | 0.50 |
| | X 34 | Xanthan Gum | 0.50 |
| B | Feel | Triheptanoin | 5.00 |
| | Imwitor 372 P | Glyceryl Stearate Citrate | 2.50 |
| | MCT Oil | Caprylic/Capric Triglyceride | 5.00 |
| | Softisan 154 | Hydrogenated Palm Oil | 1.00 |
| | Cetyl Alcohol | Cetyl Alcohol | 2.00 |
| | Shea Butter | Butyrosperum Parkii Butter | 5.00 |
| C³ | Betaine³ | - | 5.00 |
| | Demineralized Water | Aqua | 6.00 |

| | | | |
|---|---|---|---|
| ¹ 1,3-propanediol, sodium lactate, erythritol, and sodium PCA (Table 1) ² Glyceryl caprylate/magnolia officinalis bark extract (Table 2) ³ Betaine was added to cooled emulsion due to its heat sensitivity | | | |

As a reference, an o/w emulsion without a_{w}-lowering substances as well as without antimicrobial substances was tested for microbial count reductions.

An example of the effect of 28 wt% of the a_{w}-lowering substances alone without the addition of antimicrobial substances on the microbial count reduction of *P. aeruginosa, E. coli and C. albicans* is shown in Table 4.

**Table 4:**

| | **log reduction *P. aeruginosa*** | | **log reduction *E. coli*** | | **log reduction *C. albicans*** | |
|---|---|---|---|---|---|---|
| **Day** | **0 wt% a_{w}**-**lowering substances** | **28 wt% a_{w}**-**lowering substances** | **0 wt% a_{w}**-**lowering substances** | **28 wt% a_{w}**-**lowering substances** | **0 wt% a_{w}**-**lowering substances** | **28 wt% a_{w}**-**lowering substances** |
| **2** | -1.6 | 3.4 | -1.2 | 0.81 | 0.38 | 3.71 |
| **7** | -1.8 | >4.90 | -1.5 | 4.00 | 0.18 | >4.83 |
| **14** | -1.4 | >4.90 | -1.3 | >4.86 | 0.75 | >4.83 |
| **28** | -1.5 | >4.90 | -1.6 | >4.86 | 1.88 | >4.83 |

The addition of 28 wt% a_{w}-lowering substances to the o/w emulsion leads to the maximum achievable log reduction of more than 4.90 for *P. aeruginosa* on day 7, more than 4.86 for *E*. *coli* on day 14 and more than 4.83 for C. *albicans* on day 7, what is an 'A criteria pass' according to ISO 11930.

In case of the o/w-emulsion containing 28 wt% of a_{w}-lowering substances a log reduction of *S. aureus* of -0.06 on day 2, 0.69 on day 7 and 3.19 on day 14 could be obtained. In case of the o/w-emulsion containing 0.5 wt% of antimicrobial substances without any a_{w}-lowering substances a log reduction of *S. aureus* of -0.30 on day 2, 0.96 on day 7 and 3.28 on day 14 could be obtained.

In case of a synergy, wherein the o/w-emulsion containing 28 wt% of a_{w}-lowering substances in combination with 0.5 wt% of antimicrobial substances, a theoretical log reduction of *S. aureus* was calculated. What could be observed is that in the performed experiment using a combination of 28 wt% of a_{w}-lowering substances and 0.5 wt% of antimicrobial substances the theoretical maximum log reduction of *S. aureus* of 4.88 was already reached on day 7.

An o/w emulsion comprising the combination of 28 wt% of a_{w}-lowering substances and 0.5 wt% of antimicrobial substances was incubated further with *E. coli, P. aeruginosa, S. aureus, C. albicans* and A. *brasiliensis.* The microbial count reduction was measured after 2, 7 and 14 days and compared to the microbial count reduction of the reference samples each containing either 28 wt% of a_{w}-lowering substances or 0.5 wt% of antimicrobial substances. Table 5 shows an example of the values for *S. aureus.*

**Table 5:**

| **Day** | **0.5 wt% antimicrobial substances** | **28 wt% a_{w}-lowering substances** | **0.5 wt% antimicrobial substances + 28 wt% a_{w}-lowering substances (calculated)** | **0.5 wt% antimicrobial substances + 28 wt% a_{w}-lowering substances (tested)** |
|---|---|---|---|---|
| **2** | -0.30 | -0.06 | no reduction | 0.69 |
| **7** | 0.96 | 0.69 | 1.65 | >4.88 |
| **14** | 3.28 | 3.19 | >4.88 | >4.88 |

In summary, it was shown that at least three a_{w}-lowering substances in an amount of at least 10 wt% are able to inhibit microbial growth in a cosmetic or personal care composition. Furthermore it was shown that this antimicrobial effect can be further increased by adding antimicrobial substances.

### Skin barrier-enhancing effect

As test emulsion an o/w emulsion containing 28 wt% of a_{w}-lowering substances and 0.5 wt% of antimicrobial substances as described in the section above was used.

The testing method used was a randomized forearm-controlled study which is a commonly used method for testing the effect on the skin barrier of cosmetic or personal care products. Skin parameters of twelve healthy study participants with no active skin diseases, allergies or excessive hair growth at the volar forearms were measured to obtain baseline values. The participants were instructed to apply 0.2 g of the test emulsion twice daily to the randomly selected volar forearm for a period of four weeks. Participants were informed not to apply topical products at the test site for at least twelve hours prior to the measurements and to avoid water contact at the test site for at least six hours prior to the measurements.

Skin barrier function was assessed by measuring TEWL expressed in g/m2/h. SC hydration was determined by measuring the electrical capacitance of the skin surface expressed in arbitrary units (a.u.). Three measurements were performed slightly offset next to each other for each test site per assessment time, and the resulting mean value was calculated.

After four weeks of twice-daily application of the test formulation, TEWL decreased statistically significant from 6.65 ± 0.974 g/h/m2 to 5.98 ± 1.18 g/h/m2 (p < 0.05), which is a decrease of 10.1%.

In agreement with the results of the TEWL measurements, SC hydration results showed a positive effect of the test formulation on the skin barrier. After four weeks, SC hydration on the control forearm remained at baseline levels while SC hydration on the treated forearm increased statistically significant by 15.6 % from 31.9 ± 6.87 a.u. to 37.8 ± 3.04 a.u. (p < 0.01). Thus, an improvement in skin condition was observed after four weeks of application.

## Claims

1. Cosmetic or personal care composition comprising at least three a_{w} (water activity)-lowering substances in a total amount of at least 10 wt%, relative to the total weight of the composition, achieving an antimicrobial effect and resulting in a skin barrier-enhancing effect,
wherein at least 1/3 of the total amount of the at least three a_{w}-lowering substances are moisturizing agents, and
wherein the water activity of the cosmetic or personal care composition is no greater than 0.94.

2. Cosmetic or personal care composition of claim 1,
further comprising at least one antimicrobial substance in an amount achieving an additive or synergistic antimicrobial effect.

3. Cosmetic or personal care composition of claim 1 or 2,
wherein the at least three a_{w}-lowering substances are present in a total amount of at least 12 wt%, in particular of at least 15 wt%, relative to the total weight of the composition.

4. Cosmetic or personal care composition of any of the preceding claims, wherein
the at least three a_{w}-lowering substances are present in a total amount of not more than 60 wt%, preferably not more than 50 wt%, in particular not more than 40 wt%.

5. Cosmetic or personal care composition of any of the preceding claims, wherein
the at least three a_{w}-lowering substances each having a maximum molecular weight of 600 g/mol, preferably a maximum molecular weight of 500 g/mol, in particular a maximum molecular weight of 400 g/mol, and/or
a water solubility according to OECD Guideline 105 of at least 1%, preferably of at least 3%, in particular of at least 5%.

6. Cosmetic or personal care composition of any of the preceding claims, wherein
the at least three a_{w}-lowering substances are selected from the group consisting of C₃ to C₆ diols, C₄ to C₆ alditols, glycerol, C₂ to C₆ aldoses and/or ketoses, disaccharides, amino acids and/or salts thereof, peptides, betaine, carnitine, N-containing amino acid analogues like creatine, ectoine and/or L-pyrrolidone carboxylate and/or salts thereof, vitamin B or vitamin C, vitamin B or vitamin C derivatives and/or precursors, urea, allantoine, caffeine, MgSO₄, NaSO₄, NaCl, KCl, LiCl, NH₄Cl, carboxylic acids and/or derivatives, hydroxy acids, keto acids, sulfate-containing surfactants, sugar surfactants, amino acid-based surfactants, polyglycerol-based surfactants and/or mixtures thereof.

7. Cosmetic or personal care composition of any of the preceding claims, wherein
the at least three a_{w}-lowering substances comprise an organic acid and/or a C₄ to C₆ alditol and/or a vitamin B derivative and/or a salt thereof, wherein the water solubility of said substance(s) according to OECD Guideline 105 is at least 1%, preferably at least 3%, in particular at least 5%,
preferably selected from the group consisting of lactic acid, citric acid, mannitol, xylitol, sorbitol, threitol, erythritol, arabitol, nicotinic acid, niacinamide and/or mixtures therof including salts thereof.

8. Cosmetic or personal care composition of any of the preceding claims, wherein
2/3 of the total amount of the at least three a_{w}-lowering substances are moisturizing agents, in particular the total amount of the a_{w}-lowering substances are moisturizing agents.

9. Cosmetic or personal care composition of any of the preceding claims, wherein
the moisturizing agents are selected from the group consisting of glycerol, propylene glycol, 1,3-propanediol, butylene glycol, 1,2-pentanediol, 1,2-hexanediol, lactic acid, citric acid, ascorbic acid, glycolic acid, niacinamide, amino acids, PCA, ectoine, caffeine, betaine, triacetine, urea, panthenol, sorbitol, xylitol, maltitol, mannitol, erythritol, mono- and disaccharides such as fructose, glucose, lactose, sucrose, inositol, trehalose, and/or salts thereof and/or mixtures thereof.

10. Cosmetic or personal care composition of any of the preceding claims, wherein
the water activity of the cosmetic or personal care composition is no greater than 0.92, preferably no greater than 0.85, in particular no greater than 0.80.

11. Cosmetic or personal care composition of any of claims 2 to 10, wherein
the at least one antimicrobial substance is present in an amount of 0.01 to 6 wt%, preferably in an amount of 0.05 to 2 wt%, even more preferred in an amount of 0.1 to 1 wt%, in particular in an amount no greater than 0.50 wt%, relative to the total weight of the composition.

12. Cosmetic or personal care composition of any of claims 2 to 11, wherein
the at least one antimicrobial substance is selected from C₃ to C₁₀ diols, glyceryl esters of a C₈ to C₁₂ carboxylic acid, C₆ to C₁₀ hydroxamic acids, bi-(hydroxyphenyl) compounds, unsaturated and/or aromatic C₆ to C₁₀ carboxylic acids and/or salts thereof, C₄ to C₁₀ oxocarboxylic acids and/or salts thereof and/or mixtures thereof.

13. Cosmetic or personal care composition of any of the preceding claims, wherein
the at least three a_{w}-lowering substances are present in the composition in an amount effective to achieve at least one of the following criteria:
• no increase in the number of viable/live molds and yeasts against the value obtained for the inoculum at a time point of 14 days after inoculation of the composition and/or
• at least a 3 log reduction in the number of viable/live gram-negative bacteria against the value obtained for the inoculum at a time point of 14 days after inoculation of the composition.

14. Cosmetic or personal care composition of any of the preceding claims, wherein
the resulting skin barrier-enhancing effect is a decrease of the transepidermal water loss (TEWL) and/or an increase of the stratum corneum (SC) hydration, in particular a decrease of the TEWL and an increase of the SC hydration.

15. A method of inhibiting microbial growth in a cosmetic or personal care composition comprising:
• introducing at least three a_{w}-lowering substances in a total amount of at least 10 wt%, relative to the total weight of the composition, achieving an antimicrobial effect,
wherein at least 1/3 of the total amount of the at least three a_{w}-lowering substances are moisturizing agents, and
wherein the water activity of the cosmetic or personal care composition is no greater than 0.94, and
• controlling the growth of at least one microorganism,
the method preferably further comprising:
• adding at least one antimicrobial substance in an amount achieving an additive or synergistic antimicrobial effect.

16. Use of at least three a_{w}-lowering substances in a total amount of at least 10 wt%, relative to the total weight of the composition, achieving an antimicrobial effect for controlling microbial growth and resulting in a skin barrier-enhancing effect, wherein at least 1/3 of the total amount of the at least three a_{w}-lowering substances are moisturizing agents, and wherein the water activity of the cosmetic or personal care composition is no greater than 0.94.

17. Use of at least three a_{w}-lowering substances in a total amount of at least 10 wt%, relative to the total weight of the composition, and at least one antimicrobial substance in an amount achieving additive or synergistic antimicrobial effect for controlling microbial growth and resulting in a skin barrier-enhancing effect, wherein at least 1/3 of the total amount of the at least three a_{w}-lowering substances are moisturizing agents, and wherein the water activity of the cosmetic or personal care composition is no greater than 0.94.

18. The method or use of any of claims 15 to 17, wherein
the at least three a_{w}-lowering substances and/or the at least one antimicrobial substance of the cosmetic or personal care composition are preferably as defined according to one or more of any of preceding claims 2 to 14.
